# EUROPEAN PATENT APPLICATION

(11) **EP 4 847 048 A1**
(43) Date of publication of application: **02.12.2026**
(21) Application number: 25744559.3
(22) Date of filing: 21.01.2025
(51) Int. Cl.: A61K 48/00, A61K 45/00, A61P 9/04

(54) **TREATMENT OF HEART FAILURE WITH PRESERVED EJECTION FRACTION AND DRUG SCREENING**

(30) Priority: 24.01.2024 CN 202410101243
(71) Applicant: Beijing Baheal Wisart Medical Research Co., Ltd, Beijing 102300 (CN)
(72) Inventor: WANG, Li, Beijing 100037 (CN); LI, Zheng, Beijing 100037 (CN)
(74) Representative: Ghirardi, Valeria
(86) International application number: PCT/CN2025/073511
(87) International publication number: WO 2025/157118

(57) **Abstract**

The present invention relates to the field of disease treatment, and particularly to use of a reagent capable of inhibiting or knocking out *Jun* gene expression in the treatment of heart failure with preserved ejection fraction (HFpEF) and a method for drug screening.

## Description

### Cross-Reference to Related Application

The present application is based on and claims priority to the Chinese application with application number CN 202410101243.8 (filed on January 24, 2024). The disclosure of the Chinese application is incorporated into the present application in its entirety.

### Technical Field

The present invention relates to the field of disease treatment. Specifically, the present invention provides use of an agent capable of inhibiting or knocking out *Jun* gene expression in the treatment of heart failure with preserved ejection fraction (HFpEF), and a method for screening drugs.

### Background Art

Cardiovascular diseases represent one of the challenging problems in medicine. Heart failure (HF) is a major cause of death in patients with cardiovascular diseases and a significant clinical challenge, including heart failure with systolic dysfunction and heart failure with diastolic dysfunction, wherein the heart failure with diastolic dysfunction is more broadly defined as heart failure with preserved ejection fraction (HFpEF) with the development of medicine. HFpEF is a major cause of morbidity and mortality throughout the industrialized world, and its prevalence is growing at an alarming rate. HFpEF currently accounts for 50% of all HF. Patients suffering from this complex systemic disease experience typical symptoms of HF, including exercise intolerance, dyspnea, accumulation of extravascular fluid in the lungs, subcutaneous tissues, and abdominal cavity, as well as intermittent cardiovascular decompensation that often leads to emergency hospitalization for diuresis. The definition of HFpEF excludes conditions such as cardiac amyloidosis, hereditary hypertrophic cardiomyopathy, valvular disease, and other diseases with clear etiologies. HFpEF rather refers to a condition whose pathophysiology involves a multi-organ syndrome, where the heart, lungs, kidneys, skeleton, immune system, inflammation, metabolism, and other components collectively contribute to the outcome. Importantly, HFpEF is a syndrome with high morbidity and mortality. It is estimated that the global mortality rate for HF is 35%, with HFpEF accounting for more than 50% thereof.

To date, basic research on HFpEF is nearly absent, and there is a significant lack of effective drug therapies proven to alter the disease progression and prognosis of HFpEF patients, thus creating a substantial unmet clinical need.

### Contents of the Invention

The inventors of the present application have discovered for the first time that *Jun* is an important regulatory factor for the development and progression of HFpEF. Thus, the following invention is provided.

### Agent capable of inhibiting or knocking out Jun gene expression

In one aspect, the present invention provides use of an agent capable of inhibiting or knocking out *Jun* gene expression in the manufacture of a medicament for the prevention and/or treatment of heart failure with preserved ejection fraction (HFpEF). Also provided is a method for the prevention and/or treatment of heart failure with preserved ejection fraction (HFpEF), the method comprising administering an effective amount of an agent capable of inhibiting or knocking out *Jun* gene expression to a subject (e.g., a human) in need thereof. In certain embodiments, the treatment comprises at least improving cardiac diastolic function, for example, reducing diastolic parameters E/E' and/or E/A.

The agent capable of inhibiting or knocking out *Jun* gene expression described herein can disrupt the expression of *Jun* gene by any mechanism, for example, by inhibiting *Jun* expression at the RNA or protein level, such as knocking out the *Jun* gene, reducing or inhibiting gene transcription, and/or reducing or inhibiting translation of the gene's mRNA product.

In certain embodiments, the measurement of expression level can be performed at the nucleic acid level or the protein level. Methods for measuring expression at the nucleic acid level include, but are not limited to, Northern blotting, PCR, RT-PCR, or real-time RT-PCR. Methods for measuring expression at the protein level include, but are not limited to, Western blotting or polyacrylamide gel electrophoresis combined with protein staining techniques such as Coomassie Brilliant Blue or silver staining, mass spectrometry, ELISA, and the like.

### Gene Editing System

In certain embodiments, the agent capable of inhibiting or knocking out *Jun* gene expression is a gene editing system. Those skilled in the art are familiar with using gene editing systems to knock down or knock out a target gene (e.g., *Jun*).

In certain embodiments, the gene editing system can be any currently known site-specific (sequence-specific) genome editing system. In certain embodiments, the genome editing system comprises at least one site-specific nuclease, such as an RNA-guided nuclease (e.g., Cas nuclease), a zinc finger nuclease, a meganuclease, a TALE-nuclease, a recombinase, a transposase, and any combination thereof. In certain embodiments, the site-specific endonuclease targets the *Jun* gene and induces DNA breakage at the target site, leading to modification via, for example, homologous recombination (HR) or non-homologous end joining (NHEJ), resulting in disruption of the *Jun* gene.

In certain embodiments, the gene editing system is selected from the group consisting of CRISPR/Cas, TALEN, ZFN, transposon technology, PASTE technology, PE technology, base editor, and any combination thereof.

In certain embodiments, the gene editing system comprises an RNA-guided endonuclease and a guide RNA (gRNA), the gRNA comprising a guide sequence complementary to a target sequence in a target locus. In certain embodiments, the gene editing system is present on one or more vectors.

In certain embodiments, the gene editing system is a CRISPR/Cas system, which comprises a Cas effector protein (including but not limited to Cas9, Cas12a (Cpf1), Cas12b (C2c1), Cas13a (C2c2), C2c3, Cas13b) and a corresponding guide RNA (gRNA). The CRISPR/Cas system recruits the Cas enzyme protein to a target locus via a guide RNA (gRNA) to accomplish modification, wherein the gRNA comprises a guide sequence complementary to a target sequence in the target locus. In certain embodiments, the gRNA comprises a chimeric guide RNA or a single guide RNA (sgRNA). In certain embodiments, the gRNA comprises a guide sequence and a tracr pairing sequence (or direct repeat sequence). In certain embodiments, the gRNA comprises a guide sequence, a tracr pairing sequence (or direct repeat sequence), and a tracr sequence. In certain embodiments, the CRISPR-Cas system does not comprise and/or is not dependent on the presence of a tracr sequence (e.g., if the Cas protein is Cas12a).

### RNA Interference Agent

In certain embodiments, the agent capable of inhibiting or knocking out *Jun* gene expression comprises an RNA interference agent.

As used herein, the expression "RNA interference agent" refers to any agent that inhibits the expression of a target gene via the RNA interference (RNAi) mechanism. "RNA interference (RNAi)" is an evolutionarily conserved process in which the expression or introduction of RNA identical or highly similar in sequence to a target gene leads to sequence-specific degradation of messenger RNA (mRNA) transcribed from that target gene or specific post-transcriptional gene silencing (PTGS), thereby inhibiting the expression of the target gene.

Thus, those skilled in the art know that a small interfering RNA (siRNA) or microRNA (miRNA) molecule can be designed based on the sequence of the *Jun* coding gene or its transcribed mRNA by selecting a target sequence. Such siRNA or miRNA molecule can interfere with gene transcription, translation, or post-transcriptional/post-translational modification, thereby affecting protein expression.

As used herein, the siRNA refers to a small interfering RNA, which is a small RNA molecule, approximately 21 to 25 nucleotides in length, and obtained by processing with Dicer (an enzyme specific for double-stranded RNA in the RNase III family). The siRNA is a major component of siRISC and triggers the silencing of a target mRNA complementary thereto.

As used herein, the microRNA (miRNA) refers to a naturally occurring class of non-coding RNA molecules, which are approximately 21 to 25 nucleotides in length. Based on sequence complementarity to a target mRNA, they can cause the degradation of the target mRNA or inhibit its translation through specific base pairing with the target mRNA, thereby achieving post-transcriptional regulation of gene expression. The miRNA can be referenced from the miRBase database (http://microrna.sanger.ac.uk/).

Furthermore, to prolong the inhibitory effect of siRNA on target gene expression, a pair of specific oligonucleotide sequences can be designed, annealed, and cloned into a vector. The transcription product of this recombinant vector is short hairpin RNA (shRNA), which can self-fold into a stem-loop structure with a stem length of 19 to 21 bases. These 19 to 21 bases originate from a specific sequence of the mRNA of target gene. The precursor with this stem-loop structure is rapidly cleaved within the cell to form functional siRNA. The shRNA typically comprises an "antisense" sequence (or "guide" sequence) capable of forming the "stem" structure and specifically binding to the target sequence, and a "sense sequence" (or "passenger" sequence) that is reverse and complementary or partially complementary to the "antisense" sequence, as well as a "loop" sequence. The siRNA formed by cleaving such vector-expressed shRNA has characteristics of stable level and long duration in expression, thereby causing long-term inhibition of target gene expression.

In certain embodiments, the RNA interference agent is selected from small interfering RNA (siRNA), short hairpin RNA (shRNA), or microRNA (miRNA).

In certain embodiments, the RNA interference agent is used or administered in the form of an expression vector (e.g., an expression vector comprising a coding sequence for the RNA interference agent).

In certain embodiments, the RNA interference agent is an shRNA.

In certain embodiments, the shRNA comprises a first region complementary to a target sequence in the *Jun* gene or its mRNA, a second region that is reverse and complementary to the first region, and a loop region.

In certain embodiments, the shRNA comprises a nucleotide sequence as set forth in SEQ ID NO: 19. For example, the first region of the shRNA comprises or consists of the nucleotide sequence as set forth in SEQ ID NO: 19.

In certain embodiments, the shRNA comprises or consists of the nucleotide sequence as set forth in SEQ ID NO: 18.

In another aspect, the present application also provides an shRNA or its coding sequence, or a vector comprising the shRNA or its coding sequence. In certain embodiments, the shRNA comprises a nucleotide sequence as set forth in SEQ ID NO: 19. In certain embodiments, the shRNA comprises or consists of the nucleotide sequence as set forth in SEQ ID NO: 18.

### Antisense Oligonucleotide

In certain embodiments, the agent comprises an antisense oligonucleotide.

As used herein, the expression "antisense oligonucleotide" refers to a molecule that is complementary to a sense nucleic acid, e.g., complementary to the coding strand of the *Jun* gene or complementary to the mRNA sequence of the *Jun* gene. Thus, the antisense oligonucleotide can form hydrogen bonds with (i.e., anneal to) the sense nucleic acid. The antisense oligonucleotide can be complementary to the entire coding strand of the nucleic acid sequence encoding *Jun*, or only to a portion thereof, e.g., all or part of the protein coding region (or open reading frame). The antisense oligonucleotide can also be antisense to all or part of the non-coding region of the coding strand of the nucleic acid sequence encoding *Jun*. The antisense oligonucleotide can be, for example, about 5, 10, 15, 20, 25, 30, or more nucleotides in length.

The antisense oligonucleotide described above can be constructed using chemical synthesis and enzymatic ligation reactions via procedures known in the art. For example, the antisense oligonucleotide can be chemically synthesized using naturally occurring nucleotides or various modified nucleotides, wherein the modified nucleotides are designed to increase the biological stability of the molecule or increase the physical stability of the duplex formed between the antisense and sense nucleic acids, e.g., phosphorothioate derivatives and acridine-substituted nucleotides can be used. Alternatively, the antisense oligonucleotide can be produced biologically using an expression vector that comprises a target nucleic acid cloned therein such that the RNA transcribed from the inserted nucleic acid will have an antisense orientation relative to the target nucleic acid.

After obtaining the RNA interference agent or antisense oligonucleotide, the inhibitory activity of the RNA interference agent or antisense oligonucleotide on the expression level of *Jun* mRNA or protein can be further determined using the methods described above.

### Administration

The agent described herein can be administered in the form of a pharmaceutical composition formed together with a pharmaceutically acceptable carrier and/or excipient. "Pharmaceutically acceptable carrier and/or excipient" refers to a carrier and/or excipient that is pharmacologically and/or physiologically compatible with the subject and the active ingredient, and is well known in the art, and includes, but is not limited to: pH adjusting agent, surfactant, adjuvant, ionic strength enhancer, diluent, agent for maintaining osmotic pressure, agent for delaying absorption, preservative.

The agent described herein can be administered by any suitable method known in the art. Preferred routes of administration include intravenous, intraperitoneal, intramuscular, subcutaneous, spinal, or other parenteral routes of administration. Parenteral administration refers to a mode of administration typically by injection rather than enteral and topical administration, and includes, but is not limited to, intravenous, intraperitoneal, intramuscular, intra-arterial, intrathecal, intralymphatic, intralesional, intracapsular, intraorbital, intracardiac, intradermal, transtracheal, subcutaneous, subepidermal, intraarticular, subcapsular, subarachnoid, intraspinal, epidural, and intrasternal injection and infusion. Alternatively, it can be administered via non-parenteral routes, such as topical, epidermal, or mucosal routes of administration, e.g., intranasal, oral, vaginal, rectal, sublingual, or topical administration.

In certain embodiments, the agent described herein is administered to a specific organ, tissue, or cell (e.g., heart, myocardial tissue, cardiomyocyte) of a subject. For example, in certain embodiments, the agent described herein is administered in situ (e.g., cardiac in situ injection, myocardial tissue in situ injection) to the subject.

In certain embodiments, the agent described herein is administered in an organ-, tissue-, or cell-specific (e.g., heart-specific, myocardial tissue-specific, cardiomyocyte-specific) manner. For example, in certain embodiments, the agent described herein is endowed with targeting ability (e.g., ability to target heart, myocardial tissue, or cardiomyocyte), for example, endowed with the ability to enrich and/or exert activity at a target site (e.g., heart, myocardial tissue, or cardiomyocyte) of the subject.

The agent described herein can be formulated into a dosage form compatible with its intended route of administration. A preferred dosage form is an injectable solution. Such injectable solution can be a sterile injectable solution. For example, the sterile injectable solution can be prepared by incorporating the required dose of the agent described herein, and optionally, other desired ingredients (including, but not limited to, pH adjusting agent, surfactant, adjuvant, ionic strength enhancer, isotonic agent, preservative, diluent, or any combination thereof) in an appropriate solvent, followed by filter sterilization. Furthermore, the sterile injectable solution can be prepared as a sterile lyophilized powder (e.g., by vacuum drying or freeze-drying) for ease of storage and use.

The agent described herein can be formulated in dosage unit form for ease of administration. The dosage unit form refers to a physically discrete unit suitable as a single dosage for the subject to be treated; each unit contains a predetermined quantity of active ingredient calculated to produce the desired therapeutic effect in association with the required pharmaceutical carrier.

The agent described herein is administered to a subject in need thereof. In certain embodiments, the subject is a mammal. In certain embodiments, the subject is a human.

### Drug Screening

In another aspect, the present invention provides a method for screening a drug for the prevention and/or treatment of heart failure with preserved ejection fraction (HFpEF), the method comprising a step of screening for a *Jun* inhibitor. The screening step is performed *in vitro*.

In certain embodiments, the *Jun* inhibitor is capable of inhibiting or down-regulating *Jun* gene expression, or inhibiting or blocking *Jun* protein activity.

In certain embodiments, the *Jun* inhibitor is selected from the agents capable of inhibiting or knocking out *Jun* gene expression as described herein.

In certain embodiments, the *Jun* inhibitor is selected from affinity molecules capable of specifically binding to the *Jun* protein, thereby inhibiting or blocking *Jun* protein activity. In certain embodiments, the affinity molecules are selected from small molecule compounds.

In certain embodiments, the step of screening for *Jun* inhibitor comprises: detecting whether a test agent is capable of inhibiting *Jun* gene expression or *Jun* protein activity; selecting the test agent capable of inhibiting *Jun* gene expression or *Jun* protein activity as a candidate drug.

In certain embodiments, the inhibiting *Jun* gene expression comprises inhibiting at the protein level and/or the mRNA level.

In certain embodiments, the step of screening for *Jun* inhibitor comprises: (1) contacting a test agent with a cell capable of expressing the *Jun* gene; (2) determining the expression level of the *Jun* gene or the activity of the *Jun* protein; (3) comparing the determination result of step (2) with a determination result in the absence of the test agent; (4) selecting the test agent having the ability of inhibiting the gene expression or protein activity as a candidate drug. In certain embodiments, the expression level comprises protein level and/or mRNA level.

### Definition of Terms

In the present invention, unless otherwise specified, scientific and technical terms used herein have the meanings commonly understood by those skilled in the art. To better understand the present invention, the definitions and explanations of relevant terms are provided below.

As used herein, the term "heart failure with preserved ejection fraction (HFpEF)" refers to a clinical syndrome characterized by impaired ventricular relaxation and decreased compliance, leading to reduced ventricular filling and increased filling pressure, resulting in pulmonary and systemic circulation congestion, in the presence of normal or mildly reduced ventricular systolic function. HFpEF typically refers to heart failure with diastolic dysfunction. In certain embodiments, the clinical diagnostic criteria for HFpEF mainly include: (1) presence of signs and/or symptoms of heart failure; (2) cardiac imaging (mainly TTE) indicates LVEF ≥ 50%; (3) presence of objective evidence of cardiac structure and/or function abnormality consistent with left ventricular diastolic dysfunction and/or elevated left ventricular filling pressure, where the structure and/or function abnormality indicators for left ventricular diastolic dysfunction and/or elevated ventricular filling pressure mainly include: (a) mean E/e' ratio > 15; (b) left atrial volume index > 40 ml/m² (atrial fibrillation).

As used herein, the term "*Jun*" refers to the *Jun* proto-oncogene, AP-1 transcription factor subunit, also known as AP1, AP-1, cJUN, or c-Jun. *Jun* can be of human origin, or a homologous gene from other species (e.g., non-human mammals, fish, reptiles, or birds, such as rodents like mice, rats, hamsters, guinea pigs, rabbits, dogs, cats, horses, cows, sheep, pigs, goats, primates, etc.). The sequence of *Jun* is well known to those skilled in the art and can be found in various public databases. An exemplary human *Jun* gene sequence can be found at GenBank: NM_002228.4, and an exemplary human *Jun* protein sequence can be found at NCBI: NP_002219.1; an exemplary mouse *Jun* gene sequence can be found at Ensembl: ENSMUSG00000052684, NCBI Gene ID: 16476, and an exemplary mouse *Jun* protein sequence can be found at UniProtKB: P05627, NCBI: NP_034721.1.

As used herein, the term "treatment" refers to a method performed to obtain a beneficial or desired clinical result. For the purposes of the present invention, beneficial or desired clinical results include, but are not limited to, alleviating symptoms, reducing the extent of the disease, stabilizing (i.e., not worsening) the state of the disease, delaying or slowing the progression of the disease, ameliorating or alleviating the state of the disease, and alleviating symptoms (whether partially or totally), whether detectable or undetectable. Furthermore, "treatment" can also mean prolonging survival as compared to expected survival if not receiving treatment.

As used herein, the term "effective amount" refers to at least the minimum concentration required to achieve a measurable improvement in or prevention of a specific condition. An effective amount as used herein can vary depending on factors such as the patient's disease state, age, sex, and weight, and the ability of the antibody to elicit a desired response in the individual. An effective amount is also one in which any toxic or detrimental effects of the treatment are outweighed by the therapeutically beneficial effects. For prophylactic use, beneficial or desired results include results such as eliminating or reducing risk, lessening severity, or delaying the onset of the disease, including biochemical, histological, and/or behavioral symptoms of the disease, its complications, and intermediate pathological phenotypes presented during disease development. For therapeutic use, beneficial or desired results include clinical results such as reducing one or more symptoms resulting from the disease, increasing the quality of life of those suffering from the disease, decreasing the dose of other medications required to treat the disease, enhancing the effect of another medication (such as via targeting), delaying the progression of the disease, and/or prolonging survival. An effective amount can be administered in one or more administrations.

### Beneficial Effects

The inventors of the present application have discovered for the first time that *Jun* is an important regulatory factor for the development and progression of HFpEF. Inhibiting or knocking out *Jun* gene expression can effectively curb the development and progression of HFpEF, improve diastolic function, and thus be used for the prevention and treatment of HFpEF, therefore exhibiting significant clinical values. Furthermore, a method for screening a drug for the prevention and/or treatment of HFpEF is also provided.

The embodiments of the present invention will be described in detail below with reference to the accompanying drawings and examples. However, those skilled in the art will understand that the following drawings and examples are only for illustrating the present invention and are not intended to limit the scope of the present invention. Various objects and advantageous aspects of the present invention will become apparent to those skilled in the art from the accompanying drawings and the following detailed description of the preferred embodiments.

### Brief Description of the Drawings

Fig. 1 shows the construction of the HFpEF model. A: Schematic diagram of the experimental procedure; B: Detection results of diastolic function (E/A) after 5 weeks of feeding in mice; C: Detection results of diastolic function (E/E') after 5 weeks of feeding in mice.
Fig. 2 shows the relative expression level of *Jun* in cardiomyocytes after 15 weeks of HFD+L-NAME feeding, indicating high expression of *Jun* in HFpEF model mice.
Fig. 3 shows the detection of *Jun* overexpression efficiency in *Myh6-cre^{ERT2}*/*Jun*-Rosa26^{LSL/-} mice. A: Schematic diagram of the experimental procedure; B: Real-time quantitative PCR detection of overexpression level of *Jun*-OE on the fifth day after tamoxifen induction; C: Immunofluorescence staining that proved *Jun* overexpression in *Jun*-OE mice; D: Immunofluorescence staining of lung tissue and heart tissue in *Jun*-OE mice.
Fig. 4 shows the detection of diastolic function in *Myh6-cre^{ERT2}*/*Jun*-Rosa26^{LSL/-} mice. A: Schematic diagram of mouse left ventricular diastolic function detection; B: Statistical results of mouse left ventricular E/A; C: Statistical results of mouse left ventricular E/E'.
Fig. 5 shows the schematic diagram of the construction strategy for *Jun*^{flox/flox} mice.
Fig. 6 shows the electrophoresis image of PCR identification of 5' homology arm and 3' homology arm in F1 generation *Jun*^{flox/flox} mice. Numbers: F1 generation mouse numbers; WT: wild-type control; M: 1kb DNA ladder.
Fig. 7 shows the electrophoresis image of PCR identification of *Myh6-cre^{ERT2}*/*Jun*^{flox/flox} mice (2% agarose gel).
Fig. 8 shows that the knockout of *Jun* effectively alleviated the development and progression of HFpEF.
Fig. 9 shows the schematic diagram of the *Jun* knockdown plasmid structure.
Fig. 10 shows that the knockdown of *Jun* effectively alleviated the development and progression of HFpEF.

### Sequence Information

The description of sequences involved in the present application is provided in the table below.

**Table 1: Sequence Information**

| SEQ ID NO: | Description | Sequence |
|---|---|---|
| 1 | Primer F | CAGTCCAGCAATGGGCACATCA |
| 2 | Primer R | GGAAGCGTGTTCTGGCTATGCA |
| 3 | gRNA1 | ACCGAGAATTCCGTGACGACTGG |
| 4 | gRNA2 | TGAGCCAATGATAGCATGCAGGG |
| 5 | 5' homology arm sequence | gggttcttctttagggtggcataccccagcccttcccaactgagcttccagcacgatatggctaaa aagagcagaagctgggttgtggagaaaaaaaaaaaaaacaaaaaacaaaaaaaaaaaaaaaa accttctcccaggcaaagaggaagtttatgatccaagcagcagcattaacaagagcagggatct |
| | | gtttcgccttggatgaaactgctatttctttacctaagtaggaaatcagcctcttaagccacaggag gcagaagatgcacagaacccttgggcaaaagatacataattacacgagggcctagatctggga gtttctaaatacaggaacaacaaaacaaagtctatgccatcagcaccaatttccgagcaaataact aagactttaagggaaagtttgccagagtagaaaaaaatacaaaggatgtaacacagacctgag atcactttccagctgtctgtataccctgagcaagacgcatggactttctgggatagtttcctcccatg taaagtctaggtaaaaatgtatgcttcttaagataactgtgagaagaaaacagcatacttaaactgc ttggagtaactggcagataggtggtgtttagtggacgaaacttatttattactgttatcataaatcat gttaagtatgagctccttggcaaaaaaaaaaaaaaaaaaaaaaaaaaaaaaaaatagggctgcgg acagaggaactcggtttccatttgggggaagtaatggctctagacttcacagaaaccctgctcta ccctgcccccaccaaggtcagcgcttgactggtagcagccagttaccctctgcagccggcagtg aaaaagagctggcgggtttcccaccgataccagctgctattgggttctgaatactcaaaaggcaa aatatcttaaggcttcagggtacaaaccctgagctgagttctgaggcagcaaagtccaaattagaa cctttctaggtccaacttcaaacttcaagtcctaggctgaagggaggcttaagggaagagttggct cccggtcctttttctatcttgcttacgggctcctgccaaatctcaacccaggatcctccaaatcaccc cacccccaccccaccgacggcccagggtaactccgaagctggtagggaaggggctattctca gagtcaaaactaatgcctggcagcccggtgtagtggacctcattcgctctacttctagatgagacc aataaagctcagagaagttctgagatttgcccttatgccctgggagaaccaagcttgccacctcttt agtcctgaggggtggagggtggagggctgatagcagaaccttagaaaagaaataacttacatgt gtctagcgcttcctagaggccgcacaggatatgactccaccagtgagcggggaggggagttttc agcctgtttctaccagggactcctgcttaagcaccgtgacttggggcgagtccgcagctctcaag gcttgttttcccagaagcaaatcagggaggaggaagggcgggcgcagactgagtgtgcgcg ggaccgtgtgtatgtgctagcgcaggcgatcgccgaggtttcctgagtttcgaacctttatatgttg ctttcgacaccccatgggcgctgggctagcatctttcacacctagaggaggggtccctcggagg caaagcgaacacccctcccccggcacgtccagagaataaagtgttgtgccggggaccctgatg agtcggttccgaacaccgcaaatctctggtttccaggtacagcacactgcggcgcagatccttctc cgaaactaaacttccaagcatactcatcttggagggctactctcaaacccgctcaacttcaacgca cttacgtgctcccacacccagggcgcacacaacccttttaacccgcatcgctctgtcccccggaa gtgcgggggtgcggagccagcttcagtcactcagatcattcagccctttctcgggcgtgcgtgcg tgtgtgtgtgtgtgtgtgtgcccgcgcgcgggcgcgcgcctgtgtgcaaccttcactcccaccca agctctccgcccccggccactagcgcgcctcccctgctttctggatccccgcgtctcggtcccag tccccagccctgaacttgagcccgcctccgacagactccgcaagggtcctgccgaccggcagc ctccgtcactagacagccaaaccaagacgtcagcccacaatgcaccgggcgggccgggaaag acgcgccggggagggaacccgggaacacaagccgaagctgagcgcgggaggggggggg gggaggaggagaaagaagggcccaactgtaggagcgcagcggagcattacctcatcccgtga gccttcgcgggcccagagaagaatcttctagggtgaggtctccatggcgacgggtgggcccgc ccccctgagaacgacgcaagccaatgggaaagcctcggggtgacatcatgggctatttttaggg attgactggtagcagataagtGTTGAGCTCAGGCTGGATAAGGACTCA GAGTTGCACTGAGTGTGGCAGAGACAGCCTGGCAGGAGA GCGCTCAGGCAGACAGACAGACAGACGGACGGACTTGGC CAACCCGGTCGGCCGCGGACTCCGGACTGTTCATCCGTTT GTCTTCATTTTCTCACCAACTGCTTGGATCCAGCGCCCGCG GCTCCTGCACCGGTATTTTGGGGAGCATTTGGAGAGTCCC TTCTCCCGCCTTCCACGGAGAAGAAGCTCACAAGTCCGGG CGCTGCTGACAGCATCGAGAGCGGCTCCCGACCGCGCGAG GAAATAGGCGAGCGGCTACCGGCCAGCAACTTTCCTGACC CAGAGGACCGGTAACAAGTGGCCGGGAGCGAACTTTTGC AAATCTCTTCTGCGCCTTAAGGCTGCCACCGAGACTGTAA AGAAAAGGGAGAAGAGGAACCTATACTCATACCAGTTCG CACAGGCGGCTGAAGTTGGGCGAGCGCTAGCCGCGGCTGC CTAGCGTCCCCCTCCCCCTCACAGCGGAGGAGGGGACAGT TGTCGGAGGCCGGGCGGCAGAGCCCGATCGCGGGCTTCCA CCGAGAATTCC |
| 6 | Flox region (loxp-Jun fragment-loxp ) sequence | GTGACGACTGGTCAGCACCGCCGGAGAGCCGCTGTTGCTG GGACTGGTCTGCGGGCTCCAAGGAACCGCTGCTCCCCGAG AGCGCTCCGTGAGTGACCGCGACTTTTCAAAGCTCGGCAT CGCGCGGGAGCCTACCAACGTGAGTGCTAGCGGAGTCTTA ACCCTGCGCTCCCTGGAGCGAACTGGGGAGGAGGGCTCAG GGGGAAGCACTGCCGTCTGGAGCGCACGCTCCTAAACAAA CTTTGTTACAGAAGCGGGGACGCGCGGGTATCCCCCCGCT TCCCGGCGCGCTGTTGCGGCCCCGAAACTTCTGCGCACAG CCCAGGCTAACCCCGCGTGAAGTGACGGACCGTTCTATGA CTGCAAAGATGGAAACGACCTTCTACGACGATGCCCTCAA CGCCTCGTTCCTCCAGTCCGAGAGCGGTGCCTACGGCTAC AGTAACCCTAAGATCCTAAAACAGAGCATGACCTTGAACC TGGCCGACCCGGTGGGCAGTCTGAAGCCGCACCTCCGCGC CAAGAACTCGGACCTTCTCACGTCGCCCGACGTCGGGCTG CTCAAGCTGGCGTCGCCGGAGCTGGAGCGCCTGATCATCC AGTCCAGCAATGGGCACATCACCACTACACCGACCCCCAC CCAGTTCTTGTGCCCCAAGAACGTGACCGACGAGCAGGAG GGCTTCGCCGAGGGCTTCGTGCGCGCCCTGGCTGAACTGC ATAGCCAGAACACGCTTCCCAGTGTCACCTCCGCGGCACA GCCGGTCAGCGGGGCGGGCATGGTGGCTCCCGCGGTGGCC TCAGTAGCAGGCGCTGGCGGCGGTGGTGGCTACAGCGCCA GCCTGCACAGTGAGCCTCCGGTCTACGCCAACCTCAGCAA CTTCAACCCGGGTGCGCTGAGCAGCGGCGGTGGGGCGCCC TCCTATGGCGCGGCCGGGCTGGCCTTTCCCTCGCAGCCGC AGCAGCAGCAGCAGCCGCCTCAGCCGCCGCACCACTTGCC CCAACAGATCCCGGTGCAGCACCCGCGGCTGCAAGCCCTG AAGGAAGAGCCGCAGACCGTGCCGGAGATGCCGGGAGAG ACGCCGCCCCTGTCCCCTATCGACATGGAGTCTCAGGAGC GGATCAAGGCAGAGAGGAAGCGCATGAGGAACCGCATTG CCGCCTCCAAGTGCCGGAAAAGGAAGCTGGAGCGGATCG CTCGGCTAGAGGAAAAAGTGAAAACCTTGAAAGCGCAAA ACTCCGAGCTGGCATCCACGGCCAACATGCTCAGGGAACA GGTGGCACAGCTTAAGCAGAAAGTCATGAACCACGTTAAC AGTGGGTGCCAACTCATGCTAACGCAGCAGTTGCAAACGT TTTGAGAACAGACTGTCAGGGCTGAGGGGCAATGGAAGA AAAAAAATAACAGAGACAAACTTGAGAACTTGACTGGTT GCGACAGAGAAAAAAAAAGTGTCCGAGTACTGAAGCCAA GGGTACACAAGATGGACTGGGTTGCGACCTGACGGCGCCC CCAGTGTGCTGGAGTGGGAAGGACGTGGCGCGCCTGGCTT TGGCGTGGAGCCAGAGAGCAGCGGCCTATTGGCCGGCAG ACTTTGCGGACGGGCTGTGCCCGCGCGCGACCAGAACGAT GGACTTTTCGTTAACATTGACCAAGAACTGCATGGACCTA ACATTCGATCTCATTCAGTATTAAAGGGGGGTGGGAGGGG TTACAAACTGCAATAGAGACTGTAGATTGCTTCTGTAGTG CTCCTTAACACAAAGCAGGGAGGGCTGGGAAGGGGGGG AGGCTTGTAAGTGCCAGGCTAGACTGCAGATGAACTCCCC TGGCCTGCCTCTCTCAACTGTGTATGTACATATATATTTTT TTTTAATTTGATGAAAGCTGATTACTGTCAATAAACAGCTT CCTGCCTTTGTAAGTTATTCCATGTTTGTTTGTTTGGGTGTC CTGCCCAGTGTTTGTAAATAAGAGATTTGAAGCATTCTGA GTTTACCATTTGTAATAAAGTATATAATTTTTTTATGTTTTG TTTCTGAAAATTTCCAGAAAGGATATTTAAGAAAATACAA |
| | | TAAACTATTGAAAAGTAGCCCCCAACCTCTTTGCTGCATTA TCCATAGATAATGATAGCTAGATGAAGTGACAGCTGAGTG CCCAATATACTAGGGTGAAAGCTGTGTCCCCTGTCTGATT GTAGGAATAGATACCCTGCA |
| 7 | 3' homology arm sequence | TGCTATCATTGGCTCATACTCTCTCCCCCGGCAACACACAA GTCCAGACTGTACACCAGAAGATGGTGTGGTGTTTCTTAA GGCTGGAAGAAGGGCTGTTGCAAGGGGAGAGGGTCAGCC CGCTGGAAAGCAGACACTTTGGTTGAAAGCTGTATGAAGT GGCATGTGCTGTGATCATTTATAATCATAGGAAAGATTTA GTAATTAGCTGTTGATTCTCAAAGCAGGGACCCATGGAAG TTTTTAACAAAAGGTGTCTCCTTCCAACTTTGAATCTGACA ACTCCTAGAAAAAGATGACCTTTGCTTGTGCATATTTATAA TAGCGTTCGTTATCACAATAAATGTATTCAAATAATGGTTT TTAAAATCTTGAAtttcctttccctttgctaatctttgcttcctaagccgtatactggggt tttccaagtttctggggttagtgctattgcattctacacttagcaatttagaagcaaagcatttagaaa gagtagaaggagaagctggggcctggttgaactattctcccgaccctctcccaattcccacttgc caccctccccacccctcacccctccagggtgacctcacaaccaaggaagtgaccagtgtaacaa atccatgtttatttcttaatttgtttcaaaataatcaatctggcttaattattgaggcagtctggaaaagt cctattataaatactatggacagagtcacaagagagatgggcccaacactgattatataacatct gtaatccaaacctcagctctggggagactgaaaacttgaactgtgtctacagatacagctgaggtt atcaacaggtttgtattcaacaagggaataatttttgcttcgtcccttctcatccccatgagctgctgt gattatcaagcagcggcagcaatttgatatccagaaacacttcacaaaccactaattggagcctg gctgttagccctgctccagtgctcaacaggtctaggagtacctgtggtggtaaatccaaattctcac ttcctcaggagtacagctaggtctgcagaggctccgacaggcttgcgggttaccagacatactcc attgtagtaatactccttgaacaaggagttactgggagccgaggcacttttccaaacagtgaattt gctgaagtcagcaatccagaaggtgagtggatttgaccatatgtaggaatagcagagggacag taaagcactttagggtggggctctgtggaagctaaacaagagcaaagtagaaacacacacag gctagggaattgatcaccctgcatggctagactaagcctctgacaacagcacaatggagaaatg ctgcagcactccccaggtggctaaaagaaaagagaacaaaaagttcaaaggtttcaagctta tccttacactagttcctttcaactagtattttatgtacctgaaattctgactgttctaacctaaagaggc agaaaaaataataataatggtaaaaaggaaattcaataatggtaaactgcaaaagcagaaaatc agctaattactgtagacaaatgactaaatctgttatggactttcagtgtgctatactttaggtagcactt aggatgaagataatggcatataaagtcatcaattctgtcttaattatctatataaatatatgtacatatg tataattaattcatagactattaactctccattaggacagagagtttatgtaatcaatagaaatgccca catggaaatattgttatcttttctttacctcccaagagctcagtgacacaacgccctgaatcagatga aacatgttgagcatcagaagcatctttgtatggatctaggtctctgggccttttaaagttccaggctt ctgcttgtattatcctactctgccctccggccagacttgcattctggttcaaatgatcactctcacctg ttctcagaggacttgctgtcctagtctgagttcacactttgatttctttcaacaatggattacaacctgc aagtgtgagccaaaataaatcctttgcctgcccaggttgtttatggctggagagcgttgtctcagcc acagaagagtaactagaacacttggcttaagtggtaccttctctgtgaaattttcctagatggttcat ggtttccaatgttcatgtagaatgaataatcccctttggagcatcattacacttaccaaaatgtgaaa acgtgaacatctgtgaatgtctgtgtgtcgggcataacactctgtcctgtcaatcagaggacccttta agttcctagtacagtacctgacacatagtgaatgttcataaaataatattttggtacccgagtaaaca catttttttagtggaatatatttcatccatgccttagtgcgtgatacaaggtggatcaacaatagcccc cagaaggttaaacttagcctctaacagagttctgcagtccacctctcaattcgtgttccttaaagaat tatgctgttagcttcatcatacctccagccacacaaagtaacccttcctaccatcacgaacgcaagt ggtcatcttagagaaaatgaatctacctgaaacattaggcttggcagtagaggtggagaaggatc cagcaagcgcagacagtagttccagatgtctttcctagaagcaactaacttcattggtagtgacag ggagactttctgtagttagatgcatcgctggggtctagaagtcaatattcagtatttgcttgctaaga cgagaatcacactaatcaacacatgcattctttgtcattcgaaaagaattgtactgactgaacagag aaaaagaggatgctttcccagcgaccccgcccccactcccatcacccactttcagtggatcatt |
| | | catcagaaaacagatttattggtgtgcttatctgcagagaccagaaatttgaattctgtct |
| 8 | Primer I (Forward) | GGGTGCCACTTCTAGGTCAATACT |
| 9 | Primer II (Reverse) | CTCGCGCATCTTCAAATCCCTCTA |
| 10 | Primer III (Forward) | CTCGCCAAGCAAACCTCTGTCTC |
| 11 | Primer IV (Reverse) | TCCCACTTCATCCCCTTCTTTTCT |
| 12 | Primer P3 | TGAAAAGTAGCCCCCAACC |
| 13 | Primer P4 | TCCAGCCTTAAGAAACACCAC |
| 14 | Primer P5 | TCGCGGGCCCAGAGAAGAAT |
| 15 | Primer P6 | TTGGGAGAGGGTCGGGAGAATAGT |
| 16 | shjun Forward oligo | GATCCACGCAGCAGTTGCAAACGTTTCTCGAGAAACGTT TGCAACTGCTGCGTTTTTTA |
| 17 | shjun Reverse oligo | AGCTTAAAAAACGCAGCAGTTGCAAACGTTTCTCGAGA AACGTTTGCAACTGCTGCGTG |
| 18 | shRNA sequence for targeting *Jun* knockdown | ACGCAGCAGUUGCAAACGUUUCUCGAGAAACGUUUGCAA CUGCUGCGU |
| 19 | Antisense sequence in shRNA for targeting *Jun* knockdown | AAACGUUUGCAACUGCUGCGU |

### EXAMPLES

The present invention is described with reference to the following examples, which are intended to illustrate the present invention (but not to limit the present invention).

Those skilled in the art understand that the examples describe the invention by way of illustration and are not intended to limit the scope sought to be protected by the present application. The experimental methods in the examples were conventional methods unless otherwise specified. Where specific conditions are not indicated, the procedures were carried out under conventional conditions or conditions recommended by the manufacturer. The reagents or instruments used, if the manufacturer is not specified, were conventional products commercially available.

### Experimental Materials and Methods

Animals: C57BL/6N wild-type mice were purchased from Beijing Vital River Laboratory Animal Technology Co., Ltd.; *Myh6-cre^{ERT2}* mice were purchased from Shanghai Model Organisms Center, Inc., with the strain full name of C57BL/6Smoc-*Myf6^{em1(CreERT2-pA)Smoc}* (Shanghai Model Organisms Center, Inc., NM-KI-200125); *Jun-Rosa26*^{*LSL*/*LSL*} mice were purchased from Shanghai Model Organisms Center, Inc., which were Rosa26 site-specific knock-in heterozygous mice capable of conditionally overexpressing CAG-LSL-Jun-IRES-EGFP-WPRE-pA gene obtained by CRISPR/Cas9 technology; *Jun*^{flox/flox} mice were purchased from Shanghai Model Organisms Center, Inc., which were *Jun* gene flox heterozygous mice capable of conditional knockout obtained using CRISPR/Cas9 technology.

Reagents are shown in the table below:

**Table 2: Reagents**

| Reagent | Cat. No. | Supplier |
|---|---|---|
| Collagenase type II | 17101015 | Gibico |
| Polyvinylpyrrolidone (PVP) | S3596 | Selleck |
| GeneJet RNA Purification Kit | K0732 | Thermo Scientific |
| iScript TM cDNA Synthesis | 1708890 | Bio-Rad |
| iTaqUniversl SYBR Green supermix | 1725121 | Bio-Rad |
| Anti-GFP antibody | ab1218 | abcam |
| Anti-JUN antibody | ab40766 | abcam |
| Anti-ACTN2 antibody | ab9465 | abcam |

### Animal Experiment Guidelines

In this example, all animal studies were conducted in accordance with the guidelines of the Laboratory Animal Center of the Animal Care and Use Committee of Fuwai Hospital, Chinese National Cardiovascular Center. All mice were bred and housed under the same environment. Mice were randomly assigned to groups during the experiment. Echocardiographic analyses were performed by independent researchers blinded to the study objectives.

### Routine Ultrasound Detection

Mice fed under different conditions for five weeks underwent routine ultrasound detection every two weeks until the end of the fifteenth week. Specifically, transthoracic echocardiography was performed using a VisualSonics Vevo 2100 system equipped with an MS400 transducer (VisualSonics). Left ventricular ejection fraction (LVEF) and other systolic function indices were obtained from short-axis M-mode scan at the mid-ventricular level, as indicated by the presence of papillary muscles, in conscious, gently restrained mice. Apical four-chamber views were obtained in anesthetized mice for diastolic function measurements using pulsed-wave and tissue Doppler imaging at the mitral valve level. Anesthesia was induced with 2.5% isoflurane and confirmed by lack of response to firm pressure on one hind paw. During echocardiographic acquisition (under temperature-controlled conditions), isoflurane was reduced to 1.0-1.5% and adjusted to maintain heart rate within 500 beats per minute. Parameters collected included: heart rate, left ventricular end-diastolic diameter, left ventricular end-systolic diameter, end-diastolic interventricular septal thickness, left ventricular posterior wall thickness at end-diastole, left ventricular fractional shortening, LVEF, peak early diastolic transmitral Doppler flow velocity, peak late diastolic transmitral Doppler flow velocity, isovolumic relaxation time, early diastolic filling deceleration time, and peak early diastolic mitral annular velocity by tissue Doppler. At the end of the procedure, all mice recovered from anesthesia without any abnormalities. All parameters were measured at least three times, and the mean values are presented. Ultrasound detection included both systolic and diastolic function assessments.

### Example 1: Correlation between Jun expression and HFpEF

### 1.1 Induction of Heart Failure with Preserved Ejection Fraction Model

Eight- to ten-week-old male C57BL/6N wild-type mice were divided into two groups, i.e., normal group (normal diet and water), model group (high-fat diet combined with N-nitro-L-arginine methyl ester). Among them, the model group was constructed using the method described in the following literature: Gabriele G. Schiattarella et al., Nitrosative stress drives heart failure with preserved ejection fraction, https://doi.org/10.1038/s41586-019-1100-z. Specifically, high-fat diet (HFD) (60% kcal from fat (lard)) and N-nitro-L-arginine methyl ester (abbreviated as L-NAME, 0.5 g/L in drinking water) were used to induce heart failure with preserved ejection fraction, resulting in an HFpEF animal model.

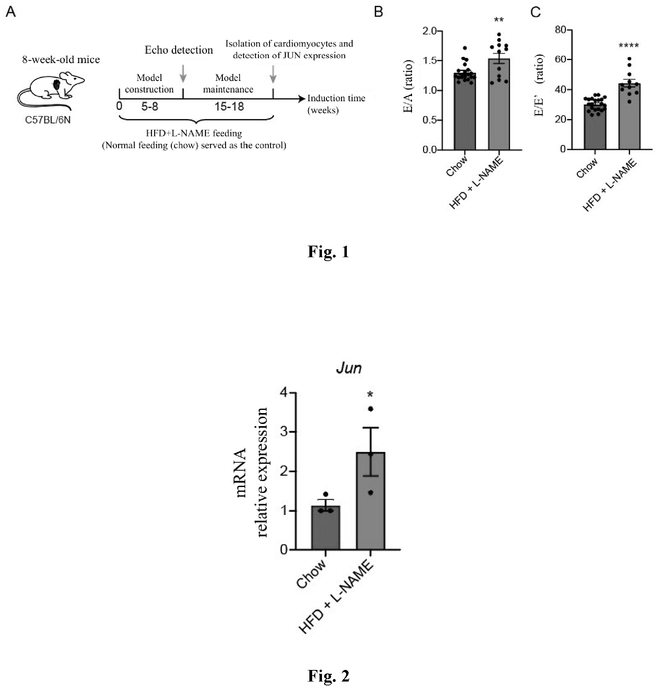

At the fifth week of model induction, the systolic function parameter LVEF of the mice did not change, while the diastolic function parameter (E/E') significantly increased, indicating the successful construction of the heart failure with preserved ejection fraction model described in the aforementioned literature. At the same time, there was no significant difference in the diastolic function parameter (E/E') between the model control group and the model treatment group at the fifth week, allowing subsequent drug administration with the same baseline, as shown in Fig. 1.

### 1.2 Correlation between Jun expression and HFpEF

At the fifteenth week of model induction, cardiomyocytes from normal mice and HFpEF animal model mice were extracted and isolated using a perfusion method, and then subjected to quantitative PCR detection. The specific operations were as follows:

### 1.2.1. Isolation of Adult Mouse Cardiomyocytes:

To isolate cardiomyocytes from adult mouse hearts, a classic perfusion method was used to isolate cardiomyocytes. Specifically, mice were injected with 100 µl of heparin sodium (1000 units in 50 ml) 20 minutes before sacrifice to prevent blood clotting in the heart, which would increase digestion difficulty during operation. Afterwards, mice were anesthetized, sacrificed, and the hearts were removed and transferred to a calcium-free solution for washing. Subsequently, the Langendorff method was used for digestion. Hearts were perfused with calcium-free solution using a Langendorff apparatus for 5 minutes, followed by digestion with an enzyme digestion solution (0.7 mg/ml type II collagenase and 0.7 mg/ml bovine serum albumin in calcium-free solution) for approximately 30 minutes. The heart was repeatedly touched at about 20 minutes, and the digestion was considered essentially complete when the heart became soft and slippery. Ventricular tissue was then collected, minced, and gently pipetted to dissociate into single cells. After settling, the supernatant was collected, undigested and adherent tissues were removed, and the cells were centrifuged at 100g for 2 minutes at 4°C to obtain a pellet of cardiomyocytes (the supernatant mostly contained non-cardiomyocytes). Cardiomyocytes were resuspended in calcium-free solution containing 10% FBS for subsequent experiments. Non-cardiomyocytes could be resuspended in culture medium or PBS for subsequent experiments. To obtain purer cardiomyocytes and non-cardiomyocytes, the cell suspension could be centrifuged (100g, 2 minutes at room temperature) three times to separate cardiomyocytes from non-cardiomyocytes. The collected cardiomyocytes were used for further experiments.

### 1.2.2. Detection by Quantitative PCR:

Total RNA was extracted from cells using the GeneJet RNA Purification Kit (Thermo Scientific, K0732), and 0.1 µg of total RNA was subjected to reverse transcription using the iScript TM cDNA Synthesis Kit (Bio-Rad, 1708890) to generate cDNA. qPCR (primer F was SEQ ID NO:1; primer R was SEQ ID NO:2) was performed using iTaqUniversl SYBR Green supermix (1725121, Bio-Rad) on an ABI Vii7 real-time system (Life Technologies, Q6). β-Actin was used for normalization. As shown in Fig. 2, compared to normal mice, significantly high expression of *Jun* was observed in the mouse HFpEF animal model. This indicates a correlation between *Jun* expression levels and HFpEF in mice, with *Jun* being highly expressed in HFpEF.

### Example 2: Induction of Heart Failure with Preserved Ejection Fraction by Jun Overexpression

### 2.1 Transgenic Mice with Jun Overexpression

*Jun* overexpressing mice *Jun-Rosa26*^{*LSL*/*LSL*} mice were crossed with cardiomyocyte-specific Cre tool mice *Myh6-cre^{ERT2}* to obtain *Myh6-cre^{ERT2}*/*Jun*-Rosa26^{LSL/-} mice, i.e., tamoxifen-inducible cardiomyocyte-specific *Jun* overexpression mice. Both *Myh6-cre^{ERT2}* mice and *Jun-Rosa26*^{*LSL*/*LSL*} mice were constructed by Shanghai Model Organisms Center, Inc.

The full name of the *Myh6-cre^{ERT2}* strain is C57BL/6Smoc-*Myf6^{em1(CreERT2-pA)Smoc}*, wherein CreERT2-pA was inserted into the start codon of the mouse Myf6 gene. When Myf6-CreERT2 was crossed with a mouse strain containing loxP-flanked sequences, induction of Cre-mediated recombination would result in deletion of the floxed sequences in the Myf6-positive cells (cardiomyocytes) of the offspring.

The construction of *Jun-Rosa26*^{*LSL*/*LSL*} mice was carried out using CRISPR/Cas9 technology. Through homologous recombination, the CAG-LSL-Jun-IRES-EGFP-WPRE-pA expression cassette was site-specifically inserted into the Rosa26 gene locus.

The *Myh6-cre^{ERT2}* mice were crossed with *Jun-Rosa26*^{*LSL*/*LSL*} mice to generate *Myh6-cre^{ERT2}*/*Jun*-Rosa26^{LSL/-} mice, which represented a conditional overexpression mouse model. In this model, tamoxifen induced the specific expression of the Cre recombinase *cre^{ERT}* in cardiomyocytes, which recognized the two LoxP sites within the LSL sequence and cleaved the stop codon between them, leading to persistent *Jun* expression in *Myh6*-positive cells.

### 2.2 Expression Efficiency Detection

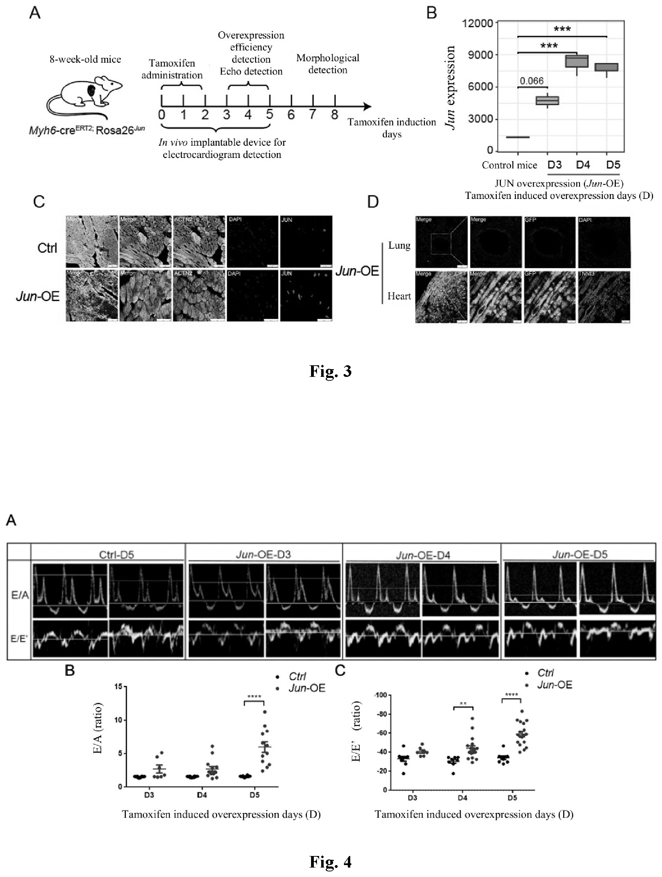

Our research found that mice died approximately 6 days after tamoxifen induction (Fig. 3, A). First, we examined the overexpression efficiency in mice. On the fifth day after tamoxifen administration, mouse cardiomyocytes were isolated, and RNA was extracted for real-time quantitative polymerase chain reaction (PCR) to detect *Jun* expression levels. The results showed that *Jun* expression in cardiomyocytes of *Myh6-cre^{ERT2}*/*Jun-Rosa26*^{*LSL*/*-*} mice was upregulated approximately tenfold compared to that of the control group (Fig. 3, B), consistent with expectations. Next, at the protein level, immunofluorescence staining also demonstrated higher *Jun* expression in heart sections of *Myh6-cre^{ERT2}*/*Jun*-Rosa26^{LSL/-} mice (Fig. 3, C). Furthermore, staining for Tag (i.e., GFP) on lung and heart sections of *Myh6-cre^{ERT2}*/*Jun-Rosa26*^{*LSL*/*-*} mice confirmed that GFP expression was detected only in heart sections (Fig. 3, D). From these data, we conclude that *Myh6-cre^{ERT2}*/*Jun-Rosa26*^{*LSL*/} could achieve cardiomyocyte-specific overexpression of *Jun*.

### 2.3 Detection of Diastolic Function Changes

Through experiments, it was demonstrated that there were no significant changes in systolic function and structural parameters after *Jun* overexpression in adult mouse cardiomyocytes. Acute myocarditis-induced death was ruled out. Enlarged atria with congestion and severe pulmonary congestion were observed. Considering this, it was hypothesized that the mice developed diastolic dysfunction heart failure, i.e., heart failure with preserved ejection fraction. Therefore, the diastolic function evaluation indices (E/E' and E/A) were assessed. As predicted, *Myh6-cre^{ERT2}*/*Jun*-Rosa26^{LSL/-} mice did not show significant changes on the third day after tamoxifen induction, but from the fourth day to the fifth day, mouse cardiac diastolic function became significantly abnormal, i.e., the E/A and E/E' values significantly increased on the fourth day and worsened on the fifth day 5 (Fig. 4, A to C). Therefore, it is concluded that overexpression of *Jun* in adult mouse cardiomyocytes led to acute heart failure with preserved ejection fraction, resulting in the death of the mice.

### Example 3: Reversion of Heart Failure with Preserved Ejection Fraction by Jun Knockout

### 3.1 Overview

Cardiomyocyte-specific Cre tool mice *Myh6-cre^{ERT2}* (Shanghai Model Organisms Center, Inc., NM-KI-200125) were crossed with floxed *Jun* gene mice *Jun*^{flox/flox} to generate *Myh6-cre^{ERT2}*/*Jun*^{flox/flox} mice. These mice could be induced by tamoxifen to achieve cardiomyocyte-specific *Jun* knockout. *Myh6-cre^{ERT2}*/*Jun*^{flox/flox} mice were referred to as *Jun*-KO mice herein.

The full name of the cardiomyocyte-specific Cre tool mouse *Myh6-cre^{ERT2}* is C57BL/6Smoc-*Myf6^{em1(CreERT2-pA)Smoc}*, wherein CreERT2-pA is inserted into the start codon of the mouse Myf6 gene. Myf6 (myogenic factor 6) is a DNA-binding protein involved in muscle development. Crossing Myf6-CreERT2 with a mouse strain in which loxP sites are inserted on both sides of the target gene (i.e., flox mice) will result, upon induction of Cre-mediated recombination, in the deletion of the gene segment between LoxP sites in the offspring's Myf6-positive cells.

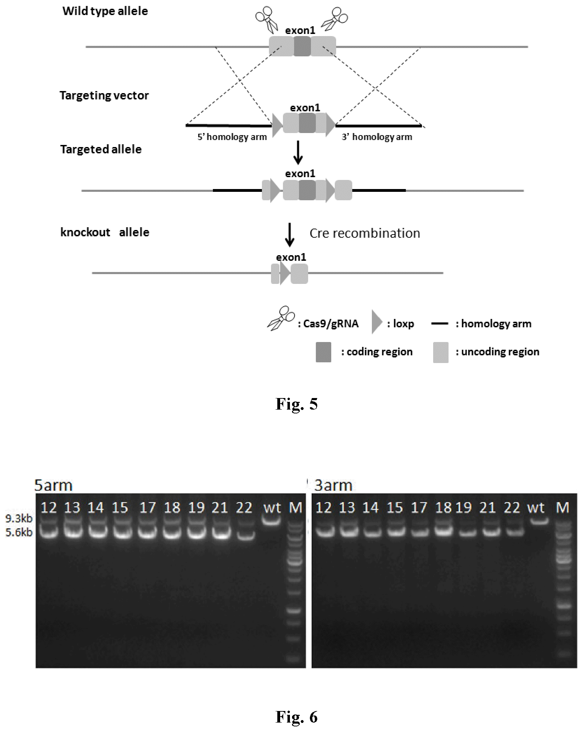

The construction strategy for floxed *Jun* gene mice *Jun*^{flox/flox} is shown schematically in Fig. 5. Based on the principle of homologous recombination, the method of zygote homologous recombination was used to perform flox modification on the *Jun* gene. The brief process is as follows: Cas9 mRNA and gRNA were obtained by *in vitro* transcription; a homologous recombination vector (donor vector) was constructed by In-Fusion cloning method, in which the vector contained a 3.1 kb 5' homology arm, a 2.2 kb flox region, and a 3.0 kb 3' homology arm. The Cas9 mRNA, gRNA, and donor vector were microinjected into zygotes of C57BL/6J mice to obtain F0 generation mice. The F0 generation mice identified as positive by PCR amplification and sequencing were mated with C57BL/6J mice to obtain 9 positive F1 generation mice. The F1 generation mice were the floxed *Jun* gene mice *Jun*^{flox/flox}.

### 3.2 Target Site and Related Sequences

Target gene name (Ensembl ID): *Jun* (ENSMUSG00000052684);
Target gene's Ensembl URL link: http://asia.ensembl.org/Mus_musculus/Gene/Summary?db=core;g=ENSMUSG00000052684;r= 4:95049034-95052222;t=ENSMUST00000107094;
Targeted transcript (Ensembl ID): Jun-201 (ENSMUST00000107094.1);
Targeted exon for flox: exon 1;
gRNA1 was SEQ ID NO: 3;
gRNA2 was SEQ ID NO: 4;
5' homology arm sequence was SEQ ID NO: 5;
flox region (loxp-Jun fragment-loxp) sequence was SEQ ID NO: 6;
3' homology arm sequence was SEQ ID NO: 7.

### 3.3 Genotype identification

Primers for 5' homology arm PCR identification: Primer I (Forward) was SEQ ID NO: 8; Primer II (Reverse) was SEQ ID NO: 9.

Primers for 3' homology arm: Primer III (Forward) was SEQ ID NO: 10; Primer IV (Reverse) was SEQ ID NO: 11.

The electrophoresis results of 5' and 3' homology arm PCR identification for F1 generation mice are shown in Fig. 6. Mice identified as PCR positive: numbers 12, 13, 14, 15, 17, 18, 19, 21, 22; all confirmed positive by sequencing.

### 3.4 Subsequent Breeding

Cardiomyocyte-specific Cre tool mice *Myh6-cre^{ERT2}* (Shanghai Model Organisms Center, Inc., NM-KI-200125) were crossed with floxed *Jun* gene mice *Jun*^{flox/flox} to generate *Myh6-cre^{ERT2}*/*Jun*^{flox/flox} mice (i.e., *Jun*-KO mice).

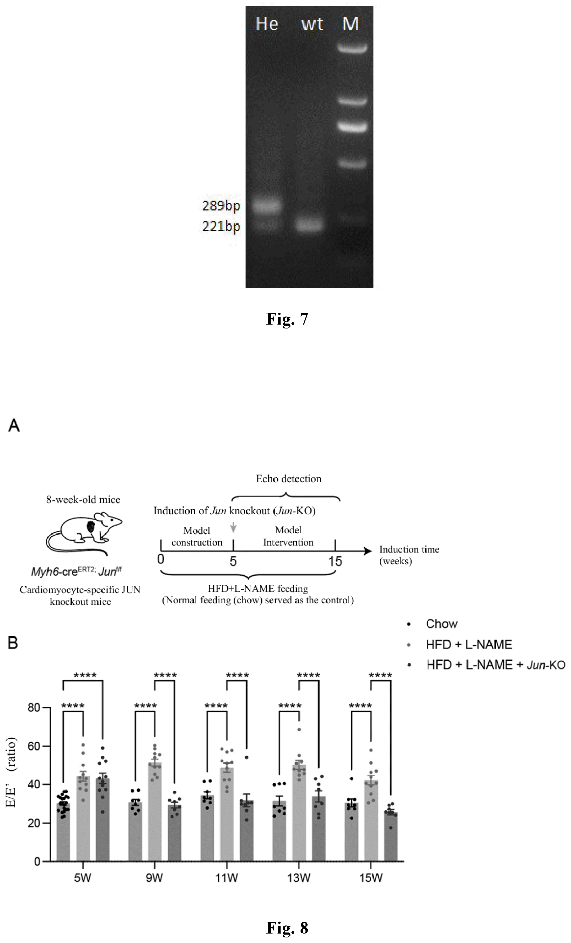

During subsequent mouse breeding, genotypes could be identified by short fragment PCR method. PCR identification conditions and primers are shown below. Exemplary results are shown in Fig. 7: WT is indicated by a single band at 221 bp; heterozygote is indicated by two bands at 221 and 289 bp; homozygote is indicated by a single band at 289 bp.

**Table 3: PCR identification conditions and primers**

| Primer | Sequence | | | | Primer type |
|---|---|---|---|---|---|
| P3 | SEQ ID NO: 12 | | | | Forward |
| P4 | SEQ ID NO: 13 | | | | Reverse |
| Reaction system for PCR | Reaction components | | | | Volume (µl) |
| | ddH₂O | | | | 14.9 |
| | 10 x Taq PCR buffer | | | | 2 |
| | 2.5 mM dNTP | | | | 1 |
| | Primer P3 (10pmol/µl) | | | | 0.5 |
| | Primer P4 (10pmol/µl) | | | | 0.5 |
| | Taq DNA polymerase* | | | | 0.1 |
| | Genomic DNA | | | | 1 |
| | Total | | | | 20 |
| | *Taq DNA Polymerase from Takara (Code number: R001A) | | | | |
| Circular reaction | Step | Temperature (°C) | Time | Notes | |
| | 1 | 94 | 5 min | | |
| | 2 | 94 | 30 sec | | |
| | 3 | 59 | 30sec | | |
| | 4 | 72 | 1 min | Repeat steps 2 to 4 for 35 cycles | |
| | 5 | 72 | 5 min | | |
| | 6 | 12 | Hold | | |

Additionally, Cre activity could be verified at the DNA level. Typically, a small piece of tissue expressing Cre was taken, genomic DNA was extracted, and the flox region was amplified by PCR. The presence or absence of the flox region was used to qualitatively determine whether Cre had functioned. PCR identification conditions and primers are shown below. Cre activity is indicated by a band at 1386 bp; no Cre activity is indicated by a band at 3638 bp; wild-type is indicated by a band at 3505 bp.

**Table 4: PCR identification conditions and primers**

| Primer | Sequence | | Primer type | | |
|---|---|---|---|---|---|
| P5 | SEQ ID NO: 14 | | Forward | | |
| P6 | SEQ ID NO: 15 | | Reverse | | |
| Reaction system for PCR | Reaction components | | Volume (µl) | | |
| | ddH₂O | | 13.2 | | |
| | GXL PCR buffer | | | | 2 |
| | 2.5 mM dNTP | | | | 2 |
| | Primer P5 (10pmol/µl) | | | | 0.5 |
| | Primer P6(10pmol/µl) | | | | 0.5 |
| | GXL DNA polymerase * | | | | 0.8 |
| | Genomic DNA | | | | 1 |
| | Total | | | | 20 |
| | * PrimeStar GXL (TaKaRa, Code No:R050A) | | | | |
| Circular reaction | Step | Temperature (°C) | Time | Notes | |
| | 1 | 95 | 3 min | | |
| | 2 | 98 | 30 sec | | |
| | 3 | 60 | 30sec | | |
| | 4 | 68 | 3 min | Repeat steps 2 to 4 for 35 cycles | |
| | 5 | 68 | 5 min | | |
| | 6 | 12 | Hold | | |

### 3.5 Experimental Procedure

After obtaining the animal model, starting from the fifth week of HFpEF model induction, the model treatment group received tamoxifen for gene knockout, while the model control group did not undergo gene knockout. Throughout the induction period, mice with normal diet and water were used as negative controls.

### 3.6 Experimental Results

The results are shown in Fig. 8. First, at the fifth week, diastolic function of the mice was assessed. Diastolic function parameters E/E' and E/A were significantly elevated, indicating diastolic dysfunction and successful construction of the model as described in the literature. Furthermore, during continuous monitoring from the fifth week to the fifteenth week, the development and progression of HFpEF were effectively suppressed in the model treatment group (undergoing *Jun* gene knockout with tamoxifen). Specifically, after *Jun* knockout, diastolic function in mice treated with a high-fat diet combined with L-NAME (HFD + 0.5g/L L-NAME) was significantly improved, and this effect persisted until the fifteenth week. In contrast, in the model control group where *Jun* was not knocked out, continuous deterioration of diastolic function was observed. Meanwhile, *Jun* expression in the model treatment group was downregulated compared to the model control group. This indicated that inhibiting or knocking out *Jun* expression could have a preventive and therapeutic effect on HFpEF in the mouse HFpEF model.

### Example 4: Reversion of Heart Failure with Preserved Ejection Fraction by Jun Knockdown

### 1. Construction of Knockdown Plasmid

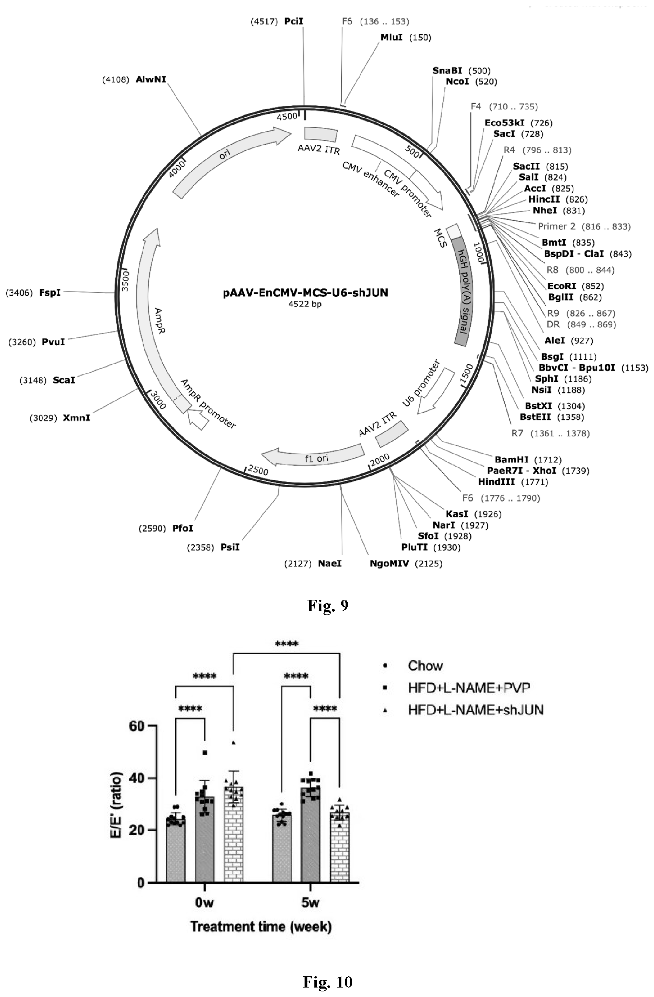

The pAAV-EnCMV-MCS-U6-shRNA plasmid (Miaoling Bio, P28262) was selected (as shown in Fig. 9). The shRNA sequence for *JUN* was ligated into the plasmid to construct a *JUN* knockdown plasmid.
shjun Forward oligo (SEQ ID NO: 16): 
GATCCACGCAGCAGTTGCAAACGTTTCTCGAGAAACGTTTGCAACTGCTGCGTT
TTTTA
shjun Reverse oligo (SEQ ID NO: 17):

AGCTTAAAAAACGCAGCAGTTGCAAACGTTTCTCGAGAAACGTTTGCAACTGCT
GCGTG

### 2. Virus Packaging and Titer Determination

### 2.1 Culture of 293T Cells

### 1. Subculture of 293T Cells

(1) Complete medium was prepared according to the formulation shown in Table 5. The complete medium and PBS (Zhongshan Jinqiao, ZLI-9062) were pre-warmed in a 37°C water bath;

**Table 5: Formulation of complete medium (50 mL)**

| Reagent required | Volume required |
|---|---|
| DMEM (high glucose) (Gibco, 11965118) | 44.5mL |
| Fetal Bovine Serum (FBS) (PAN-Biotech, P30-3306) | 5mL |
| Penicillin-Streptomycin (P/S) (Gibco, 15140122) | 500µL |

(2) Taking a 10 cm cell culture dish as an example, when the cells (Chinese Academy of Sciences, GNHu44) were cultured to approximately 70% to 80% confluence, they were ready for passaging. The cell culture medium was then removed using a suction pump.
(3) After adding 1 mL of the pre-warmed PBS, the culture dish was gently tilted back and forth at a 45° angle to allow the PBS to cover all the cells, washing away dead cells and debris. The PBS was then removed using a suction pump.
(4) After adding 1 mL of 0.25% trypsin (Gibco, 25200056), the culture dish was gently tilted back and forth at a 45° angle to allow the trypsin to cover all the cells for cell dissociation. As soon as cell detachment was observed, the trypsin was immediately removed using a suction pump.
(5) After adding 2 mL of the pre-warmed complete medium for neutralization, the culture dish was gently tilted back and forth at a 45° angle to allow the medium to cover all the cells. The 293T cells were then gently pipetted off to form a single-cell suspension.
(6) The 293T cell suspension was transferred into a 15 mL centrifuge tube and centrifuged at 1000 rpm for 3 minutes at room temperature. The supernatant was then discarded.
(7) After adding 2 mL of the pre-warmed complete medium to resuspend the cell pellet, one-third of the cell suspension was replated into a new cell culture dish. Then, 7 mL of complete medium was added and thoroughly mixed by shaking. The cells were cultured in a cell culture incubator maintained at 37°C with 5% CO₂ and 95% humidity.

### 2. Cryopreservation of 293T Cells

(1) The complete medium and PBS were preheated in a 37°C water bath.
(2) Taking a 10 cm cell culture dish as an example, when the cells were cultured to approximately 70% to 80% confluence, they were ready for passaging. The cell culture medium was removed by a suction pump.
(3) 1 mL of the preheated PBS was added, and the dish was gently tilted back and forth at a 45° angle to allow the PBS to cover all the cells, washing away dead cells and debris. The PBS was then removed by a suction pump.
(4) 1 mL of 0.25% trypsin was added, and the dish was gently tilted back and forth at a 45° angle to allow the trypsin to cover all the cells for cell dissociation. As soon as cell detachment was observed, the trypsin was immediately removed by a suction pump.
(5) 2 mL of the preheated complete medium was added for neutralization, and the dish was gently tilted back and forth at a 45° angle to allow the medium to cover all the cells. The 293T cells were gently pipetted off to form a single-cell suspension.
(6) The 293T cell suspension was transferred to a 15 mL centrifuge tube and centrifuged at 1000 rpm for 3 minutes at room temperature. The supernatant was then discarded.
(7) 2 mL of cryopreservation medium was added to resuspend the cell pellet. 1 mL of the cell suspension was aliquoted into cryovials, which were then stored in a freezing container at -80°C for slow cooling.
(8) On the next day, the cryovials were transferred to a -196°C liquid nitrogen tank for long-term storage.

### 2.2 AAV Packaging

(1) 293T cells were seeded into 15 cm cell culture dishes using 293T cell culture medium and were incubated in a cell culture incubator at 37°C with 5% CO₂ and 95% humidity.
(2) One day in advance, the cells were passaged at a ratio of 2:3.
(3) Two hours before transfection, the medium in each dish was replaced with 18 mL of antibiotic-free, low-serum medium (prepared according to the formulation shown in Table 6), and the cells were incubated in a cell culture incubator at 37°C with 5% CO₂ and 95% humidity. When the cell density reached 80% to 90% (noting the difference between the central and peripheral regions), adeno-associated virus (AAV) packaging was performed.

**Table 6: Formulation of low-serum, antibiotic-free medium (50 mL)**

| Reagent required | Volume required |
|---|---|
| DMEM (High Glucose) | 48.5mL |
| Fetal Bovine Serum (FBS) | 1mL |
| Penicillin-Streptomycin (P/S) | 500µL |

(4) The AAV packaging system was prepared and used immediately: pAAV-ITR, pAAV-2/9n (Miaoling Bio, P12267), and pAdDeltaF6 (Miaoling Bio, P10945) were mixed at a molar ratio of 1:1:1. The amount of each plasmid in a total of 80 µg of all plasmids was calculated according to the molar ratio. 3 mL of Opti-MEM (Gibco, 31985070) was then added and the mixture was allowed to stand for 5 min to ensure thorough mixing.
(5) The transfection reagent was prepared using PEI at 3 times the DNA amount. 240 µL of PEI (Polysciences, 24765-1) was added to 3 mL of Opti-MEM, and the tube was tilted at a 45° angle and gently inverted back and forth for mixing well. The mixture was then allowed to stand for 5 min for thorough mixing.
(6) After 5 min of mixing, the plasmid mixture was added to the PEI liposome mixture. It was gently mixed by inverting the tube at a 45° angle several times and then allowed to stand for 15 min to obtain the plasmid-PEI mixture. Note: Pipetting and vibration were avoided, as PEI served as the lipid component and harsh shaking was known to potentially disrupt its structure.
(7) Excess antibiotic-free medium was pipetted from the 293T cell culture dishes, leaving approximately 14 mL per dish. About 6 mL of the plasmid-PEI mixture was slowly added along the sidewall of each dish, bringing the total volume to approximately 20 mL per dish. The dishes were gently tilted to mix the contents and then incubated in a cell culture incubator at 37°C with 5% CO₂ and 95% humidity. After 24 hours of plasmid transfection, the medium was replaced with antibiotic-free, low-serum medium (20 mL per 15 cm dish), and the cells were cultured in the incubator at 37°C with 5% CO₂ and 95% humidity.
(8) After changing the medium, the cells were cultured for an additional 48 to 72 hours. When cytopathic effect (CPE) was observed, the 293T cells were harvested by gently pipetting the cells off the dish. The culture medium and cell pellet were collected together into 50 mL centrifuge tubes. After centrifugation at 2000 rpm for 10 min at 4°C, the supernatant was removed, and the cell pellet was frozen at -80°C until lysis for AAV extraction.

### 2.3 AAV Extraction

(1) The frozen pellet of 293T cells obtained after AAV packaging was taken out from the -80°C freezer and resuspended in 9 mL of PBS (autoclaved);
(2) 1 mL of Lysis Buffer (0.5% sodium deoxycholate + 0.1% SDS + 1% Triton^{TM} X-100) and PF68 (MP Biomedicals, 092750016) at a final concentration of 0.001% were added; after mixing, the suspension was incubated in a 37°C water bath for 15 min (Sodium deoxycholate (Sigma-Aldrich, D6750-25G); SDS (VWR, 0227-1KG); Triton^{TM} X-100 (Sigma-Aldrich, T8787-100ML));
(3) The aforementioned cell suspension was subjected to repeated freeze-thaw lysis using -196°C liquid nitrogen and a 37°C water bath, and this freeze-thaw cycle was repeated four times;
(4) Benzonase Nuclease (Novoprotein Scientific (Shanghai) Inc., M046-01B) was added to a final concentration of 500 U/mL, together with MgCl₂ to a final concentration of 2 mM; and the mixture was then incubated in a 37°C water bath for 45 min (until nucleic acid fragments disappeared);
(5) 4 mL of 5 M NaCl was added, and the mixture was shaken for 30 s (to prevent AAV loss);
(6) The AAV solution was centrifuged at 4000 rpm for 10 min at 4°C. The supernatant was collected into a new centrifuge tube and centrifuged again at 4000 rpm for 10 min at 4°C. The supernatant was then collected into a new centrifuge tube and stored in a 4°C refrigerator until density gradient centrifugation. This centrifugation process was repeated multiple times until almost no pellet remained in the supernatant;
(7) Density gradient centrifugation was performed in a biosafety cabinet (Thermo): Using a 39 mL ultracentrifuge tube, iodixanol (Sigma, D1556-250ML) gradient solutions (formulations shown in Table 7) were added sequentially with a 10 mL syringe (1 mL syringe needle). Specifically, 5.4 mL of 60% solution (which could be added relatively quickly), 6.5 mL of 40% solution, 6.5 mL of 25% solution, and 8 mL of 15% solution were sequentially added. To prevent layer mixing, the tube was tilted during addition, and each solution was injected slowly; this precaution was particularly critical for the 15% layer, as its density closely resembled that of the 25% layer, making them prone to mixing. Finally, 12 mL of viral supernatant was added, and the remaining volume was filled up with PBS. Bubble formation was avoided during injection, for example, 7 mL was drawn to prevent disturbance and bubble retention in the tube for a 6.5 mL solution, and the neck of the ultracentrifuge tube was kept free of liquid);

**Table 7: Formulation of iodixanol gradient solutions (50 mL)**

| Iodixanol gradient solution | 15% | 25% | 40% | 58% |
|---|---|---|---|---|
| Iodixanol 60% solution | 12.5mL | 20.8mL | 33.3mL | 48.2mL |
| 10x gradient buffer | 5mL | 5mL | 5mL | 1.7mL |
| NaCl (5M) | 10mL | 0mL | 0mL | 0mL |
| ddH₂O | 22.5mL | 24.1mL | 11.7mL | 0mL |
| Phenol red (5mg/mL) | 0µL | 100µL | 0µL | 100µL |

wherein, the 10× gradient buffer was prepared according to Table 8.

**Table 8: Formulation of 10× gradient buffer (100 mL)**

| Reagent required | Volume required |
|---|---|
| 1M Tris (pH7.6) | 10mL |
| NaCl (5M) | 30mL |
| MgCl₂ (1M) | 10mL |
| ddH₂O | 50mL |

(8) The ultracentrifuge tube was heat-sealed and centrifuged in an ultracentrifuge at 300,000 g for 2.5 h at 10°C;
(9) The ultracentrifuge tube was taken out and fixed on an iron stand, and a 10 mL syringe needle was inserted at a 45° angle into the upper part of the ultracentrifuge tube for air venting;
(10) A 1 mL syringe needle equipped with a 10 mL syringe was advanced along the inner wall of the ultracentrifuge tube and inserted vertically at the interface between the 40% and 60% gradient solutions. The AAV salt solution in the 40% gradient layer was withdrawn, collected into a centrifuge tube, and stored in a 4°C refrigerator.

### 2.4 Desalting and Purification of AAV

(1) The ultrafiltration centrifuge tube was loaded with PBS containing 1% PF68 for column equilibration; after 3 minutes, centrifugation was carried out at 3000g for 3 minutes, and the waste liquid was discarded;
(2) The ultrafiltration centrifuge tube was loaded with PBS containing 0.1% PF68 for column equilibration; after 3 minutes, centrifugation was carried out at 3000g for 3 minutes, and the waste liquid was removed;
(3) The ultrafiltration centrifuge tube was loaded with PBS containing 0.01% PF68 for column equilibration; after 3 minutes, centrifugation was carried out at 3000g for 3 minutes, and the waste liquid was discarded;
(4) Four volumes of PBS (autoclaved) were added to the AAV salt solution containing iodixanol to reduce viscosity; after being mixed thoroughly, the solution was loaded into the ultrafiltration centrifuge tube and centrifuged for desalting at 3000g and 4°C for 10 minutes; and centrifugation was carried out for several times until only 1 mL of the salt solution remained;
(5) Four volumes of PBS (autoclaved) were further added to the AAV salt solution containing iodixanol, then desalting was carried out by centrifugation at 3000g and 4°C for 20 to 30 minutes, until 250 µL of the AAV solution remained (the concentrate volume was checked every 5 minutes);
(6) The ultrafiltration centrifuge membrane was rinsed with 250 µL (or 100 µL if the virus amount was low) of 0.002% PF68 in PBS (autoclaved); and the AAV solution was then aliquoted into PCR tubes at 50 µL per tube and cryopreserved in a refrigerator at -80°C.

### 2.5 AAV Titer Determination

### (1) Removal of free DNA molecules from AAV samples

5 µL of AAV was taken and added into 45 µL of PBS buffer, resulting in a 10-fold dilution of the sample. The sample was further diluted with PBS buffer by factors of 10², 10³, 10⁴, and 10⁵ to prepare virus dilutions, and the reaction system was prepared according to Table 9 below.

**Table 9: Reaction system**

| Reagent required | Volume required |
|---|---|
| Virus dilution | 4µL |
| ddH₂O | 4µL |
| DNase I (Thermo Scientific, EN0521) | 1µL |
| 10x DNase I Buffer (Thermo Scientific, EN0521) | 1µL |

The prepared reaction system is incubated in a PCR thermal cycler at 37°C for 30 minutes to degrade free DNA, then at 95°C for 5 minutes to inactivate DNase I.

### (2) Removal of AAV capsid protein

1 µL of Proteinase K (Solarbio, P9460) (5 µg/µL) was added to each reaction system, and incubated at 37°C for 30 minutes to fully remove AAV capsid protein.

30 µL of ddH₂O was added to each reaction system for dilution, and incubated at 95°C for 5 minutes to inactivate Proteinase K.

### (3) Dilution of adeno-associated virus titer determination standard

The AAV titer determination standard was serially diluted to obtain 10⁷, 10⁶, 10⁵, 10⁴, 10³, 10², and 10-fold dilutions.

### (4) Real-time PCR reaction

The AAV nucleic acid sample from the above reaction system and the serially diluted standard were taken, and the reaction system shown in Table 10 was prepared for Real-time PCR detection.

**Table 10: Reaction system for Real-time PCR**

| Reagent required | Volume required |
|---|---|
| SYBR Green Supermix | 5µL |
| Primer Forward (4µM) | 1µL |
| Primer Reverse (4µM) | 1µL |
| cDNA | 3µL |

The reaction was carried out using a Real-Time PCR system (e.g., ABI Quant Studio 6) according to the program shown in Table 11.

**Table 11: Reaction program for PCR**

| Temperature (°C) | Reaction time | |
|---|---|---|
| 50°C | 2min | |
| 95°C | 3min | |
| 95°C | 15s | 40cycles |
| 60°C | 15s | |
| 72°C | 15s+plate read | |
| 95°C | 15s | |
| 60°C | 1min | |
| 95°C | 15s | |

### 3. Administration of adeno-associated virus for Jun knockdown

(1) The purified adeno-associated virus obtained from the above steps was administered by cardiac *in situ* injection at a dose of 5× 10¹¹ copies.
(2) After the injection, the mice were placed on a heating pad until full recovery from anesthesia and subsequently returned to their original cages for continued housing and subsequent analyses.

### 4. Experimental Results

The results are shown in Fig. 10. First, at week 8 (the 0^{th} week after knockdown treatment), diastolic function of the mice was assessed. Diastolic function parameters E/E' and E/A were significantly elevated, indicating diastolic dysfunction and successful construction of the model as described in the literature. Furthermore, during a continuous 13-week monitoring period (including 5 weeks of knockdown treatment), the development and progression of HFpEF in the model treatment group (using shRNA for knockdown of *Jun* gene) were effectively suppressed. Specifically, after *Jun* knockdown, diastolic function was significantly improved in mice treated with a high-fat diet combined with L-NAME (HFD + 0.5 g/L L-NAME). In contrast, mice in the model control group, where *Jun* was not knocked down, exhibited continuous deterioration of diastolic function. These findings indicated that inhibiting or knocking down *Jun* expression could exert both preventive and therapeutic effects on HFpEF in the mouse HFpEF model.

Although the specific embodiments of the present invention have been described in detail, those skilled in the art will appreciate that various modifications and changes can be made to the details based on all the teachings that have been published, and these changes are within the scope sought to be protected by the present invention. The full scope of the present invention is given by the appended claims and any equivalents thereof.

## Claims

1. Use of an agent capable of inhibiting or knocking out *Jun* gene expression in the manufacture of a medicament for the prevention and/or treatment of heart failure with preserved ejection fraction (HFpEF).

2. The use according to claim 1, wherein the agent is a gene editing system.

3. The use according to claim 2, wherein the gene editing system comprises at least one site-specific nuclease.

4. The use according to claim 3, wherein the site-specific nuclease is selected from the group consisting of RNA-guided nuclease (e.g., Cas nuclease), zinc finger nuclease, meganuclease, TALE-nuclease, recombinase (e.g., Cre recombinase), transposase, and any combination thereof.

5. The use according to claim 1, wherein the agent is an RNA interference agent or an antisense oligonucleotide.

6. The use according to claim 5, wherein the RNA interference agent is siRNA, shRNA, or miRNA.

7. The use according to any one of claims 1 to 6, wherein the agent is capable of knocking out *Jun* gene, reducing or inhibiting the transcription of *Jun* gene, and/or reducing or inhibiting the translation of *Jun* mRNA product.

8. A method for screening a drug for the prevention and/or treatment of heart failure with preserved ejection fraction (HFpEF), the method comprising a step of screening for a *Jun* inhibitor.

9. The method according to claim 8, wherein the *Jun* inhibitor is capable of inhibiting or down-regulating *Jun* gene expression, or inhibiting or blocking *Jun* protein activity.

10. The method according to claim 8, wherein the step of screening for a *Jun* inhibitor comprises: detecting whether a test agent is capable of inhibiting *Jun* gene expression or *Jun* protein activity; selecting the test agent capable of inhibiting *Jun* gene expression or *Jun* protein activity as a candidate drug.

11. The method according to claim 9 or 10, wherein the inhibiting *Jun* gene expression comprises inhibiting at the protein level and/or the mRNA level.

12. A method for the prevention and/or treatment of heart failure with preserved ejection fraction (HFpEF), comprising administering an effective amount of an agent capable of inhibiting or knocking out *Jun* gene expression to a subject (e.g., a human) in need thereof;
preferably, the agent is as defined in any one of claims 2 to 7.
